# EUROPEAN PATENT APPLICATION

(11) **EP 4 760 248 A1**
(43) Date of publication of application: **17.06.2026**
(21) Application number: 25215728.4
(22) Date of filing: 13.11.2025
(51) Int. Cl.: G01N 27/12, G01N 33/00

(54) **SYSTEMS, APPARATUSES, AND METHODS FOR GAS SENSING**

(30) Priority: 13.12.2024 IN 202411098689
(71) Applicant: Honeywell International Inc., Charlotte, NC 28202 (US)
(72) Inventor: JAYAKUMAR, Prince Ashwin Kumar Anburaj, Charlotte, 28202 (US); KASHYAP, Pavan Ramachandravitthal, Charlotte, 28202 (US); BECK, Scott E., Charlotte, 28202 (US); XU, Miao, Charlotte, 28202 (US)
(74) Representative: Haseltine Lake Kempner LLP

(57) **Abstract**

Systems, apparatuses, and methods for gas sensing are provided. A gas sensor may be a 3D MEMS gas sensor and include a plurality of electrodes and a plurality of polymer coatings. Each of the plurality of polymer coatings is deposited on top of one of one of the plurality of electrodes, and each of the plurality of polymer coatings has a different coating depth. The polymer coatings may change impedance in various concentrations of a target gas. The gas sensor may transmit a signal through the one or more polymer coatings and generate an output signal based on a concentration of the target gas.

## Description

### TECHNOLOGICAL FIELD

Example embodiments of the present disclosure relate generally to gas sensors, particularly to a sense element for gas sensing.

### BACKGROUND

Gas sensors are used in multiple applications. Battery safety is an application that may utilize a gas sensor to detect a gas generated by a battery or battery cell. A battery or battery cell may generate gas when the it is damaged or abused. A gas sensor may be used to detect the damage or abuse.

The inventors have identified numerous areas of improvement in the existing technologies and processes, which are the subjects of embodiments described herein. Through applied effort, ingenuity, and innovation, many of these deficiencies, challenges, and problems have been solved by developing solutions that are included in embodiments of the present disclosure, some examples of which are described in detail herein.

### BRIEF SUMMARY

In accordance with some embodiments of the present disclosure, an example 3D MEMS gas sensor is provided. The 3D MEMS gas sensor comprises: a plurality of electrodes; and a plurality of polymer coatings, wherein each of the plurality of polymer coatings is deposited on top of one of one of the plurality of electrodes, and wherein each of the plurality of polymer coatings has a different coating depth.

In some embodiments, each polymer coating of the plurality polymer coatings is comprised of a polymer that changes impedance based on a concentration of a first gas.

In some embodiments, the 3D MEMS gas sensor is configured to generate an output signal that varies based on the concentration of the first gas.

In some embodiments, the first gas is a carbonate gas.

In some embodiments, each of the plurality of polymer coatings is separated from one or more adjacent polymer coatings by one or more sidewalls.

In some embodiments, each of the one or more sidewalls is comprised of a dielectric material.

In some embodiments, the 3D MEMS gas sensor of further comprising a plurality of amplifiers, and wherein each of the plurality of electrodes is electrically connected to one of the plurality of amplifiers, and wherein each of the plurality of amplifiers is configured to amplify an electrode signal received from the associated electrode.

In some embodiments, a first amplifiers of the plurality of amplifiers is electrically connected to a second amplifier of the plurality of amplifiers to provide a supply voltage to the second amplifier.

In some embodiments, each of the plurality of amplifiers comprise an amplifier output; and each of the amplifier outputs is electrically connected to a summation circuit configured to generate a summation circuit output signal based on one or more amplifier output signals received from the plurality of amplifiers.

In some embodiments, the summation circuit is configured to generate a summation circuit output signal of a voltage associated with the concentration of the first gas.

In accordance with some embodiments of the present disclosure, an example method of gas sensing is provided. The method of gas sensing comprises: providing a 3D MEMS gas sensor comprising: a plurality of electrodes; and a plurality of polymer coatings, wherein each of the plurality of polymer coatings is deposited on top of one of one of the plurality of electrodes, and wherein each of the plurality of polymer coatings has a different coating depth; exposing the 3D MEMS gas sensor to a first gas; and generating an output signal when a concentration of the first gas is above a first threshold.

In some embodiments, each polymer coating of the plurality polymer coatings is comprised of a polymer that changes impedance based on the concentration of the first gas.

In some embodiments, the output signal changes when the concentration of the first gas is above a second threshold.

In some embodiments, the first gas is a carbonate gas.

In some embodiments, each of the plurality of polymer coatings is separated from one or more adjacent polymer coatings by one or more sidewalls.

In some embodiments, each of the one or more sidewalls is comprised of a dielectric material

In some embodiments, the 3D MEMS gas sensor further comprises: a plurality of amplifiers; each of the plurality of electrodes is electrically connected to one of the plurality of amplifiers; and each of the plurality of amplifiers is configured to amplify an electrode signal received from the associated electrode.

In some embodiments, a first amplifiers of the plurality of amplifiers is electrically connected to a second amplifier of the plurality of amplifiers to provide a supply voltage to the second amplifier.

In some embodiments, each of the plurality of amplifiers comprise an amplifier output; and each of the amplifier outputs is electrically connected to a summation circuit configured to generate the output signal based on one or more amplifier output signals received from the plurality of amplifiers.

In some embodiments, the summation circuit is configured to generate an output signal of a voltage associated with the concentration of the first gas.

The above summary is provided merely for purposes of summarizing some example embodiments to provide a basic understanding of some aspects of the disclosure. Accordingly, it will be appreciated that the above-described embodiments are merely examples and should not be construed to narrow the scope or spirit of the disclosure in any way. It will also be appreciated that the scope of the disclosure encompasses many potential embodiments in addition to those here summarized, some of which will be further described below.

### BRIEF DESCRIPTION OF THE DRAWINGS

Having thus described certain example embodiments of the present disclosure in general terms, reference will now be made to the accompanying drawings, which are not necessarily drawn to scale, and wherein:
FIG. 1A illustrates a cross-section view of an exemplary block diagram of a gas sensor in accordance with one or more embodiments of the present disclosure;
FIG. 1B illustrates a cross-section view of a second exemplary block diagram of a gas sensor in accordance with one or more embodiments of the present disclosure;
FIG. 2 illustrates a top view of an exemplary block diagram of a gas sensor in accordance with one or more embodiments of the present disclosure;
FIG. 3 illustrates a flowchart of example operations for sensing a gas in accordance with one or more embodiments of the present disclosure; and
FIG. 4 illustrates an exemplary block diagram of a device in accordance with one or more embodiments of the present disclosure.

### DETAILED DESCRIPTION

Some embodiments of the present disclosure will now be described more fully herein with reference to the accompanying drawings, in which some, but not all, embodiments of the disclosure are shown. Indeed, various embodiments of the disclosure may be embodied in many different forms and should not be construed as limited to the embodiments set forth herein; rather, these embodiments are provided so that this disclosure will satisfy applicable legal requirements. Like reference numerals refer to like elements throughout.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in various embodiments," "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments or it may be excluded.

The use of the term "circuitry" as used herein with respect to components of a system or an apparatus should be understood to include particular hardware configured to perform the functions associated with the particular circuitry as described herein. The term "circuitry" should be understood broadly to include hardware and, in some embodiments, software for configuring the hardware. For example, in some embodiments, "circuitry" may include processing circuitry, communications circuitry, input/output circuitry, and the like. In some embodiments, other elements may provide or supplement the functionality of particular circuitry.

### Overview

Various embodiments of the present disclosure are directed to improved gas sensors.

Gas sensors may be used to sense the presence and/or concentration of a gas. A type of gas sensor is a microelectromechanical systems (MEMS) gas sensor that utilizes mechanical and electrical components to sense the presence and/or concentration of a gas. Various embodiments described herein are MEMS gas sensors. In various embodiments, a gas sensor may be fabricated out of silicon using semiconductor fabricating techniques. The sensor may also be constructed through non-MEMS methods, such as using a glass substrate instead of silicon and performing a PVDF coating.

In an exemplary application, a gas sensor is used in a battery application to sense the presence and/or measure the concentration of a gas generated by one or more batteries. For example, an embodiment may measure the concentration of carbonate gas or vapors. The gas sensor may be a 3 dimensional (3D) gas sensor that senses gas based on 3D configuration of the gas sensor as described herein. The 3D configuration refers to a polymer coating having a surface area and a depth.

Various embodiments of the present disclosure include a gas sensor that includes a plurality of electrodes that are each located under its own polymer coating. Each of the polymer coatings has a different depth and, thus, each of the electrodes is at a different depth.

The polymer coatings are of a polymer that changes impedance in the presence of a target gas. In various embodiments, the depth of a polymer deposited on top of an electrode is associated with how much of a concentration of gas is needed to change to the impedance of the polymer coating. For example, a shallower polymer coating will change its impedance in the presence of a lower concentration while a deeper polymer coating will require a great concentration to change its impedance. In various embodiments, the polymer may absorb the gas over time and the greater the concentration of gas may cause a greater absorption. As the polymer saturates with the target gas then the impedance may change. The change in impedance of a polymer coating may be detected in the gas sensor by transmitting an electrical signal from the top of a polymer coating to an electrode under the polymer coating. Utilizing multiple polymer coatings at different depths allows for the gas sensor to detect the presence of gas at one or more concentrations.

The gas sensor has a sensing surface and the top of each of the polymer coatings are on the sensing surface. When the polymer coatings on the sensing surface of the gas sensor are exposed to a target gas then the impedance of the polymer coatings may change.

In various embodiments, the change in impedance of the polymer is a step response that may be nonlinear. The nonlinear response of the polymer to a concentration of a target gas may allow for each respective polymer coating at each respective depth and the associated electrode to generate an electrode signal associated with a particular concentration of the target gas. In various embodiments, the gas sensor may be configured and/or calibrated to generate a gas sensor signal associated with the concentration of the target gas.

In various embodiments, the polymer may change its impedance in the presence of carbonate gas. The gas sensor may be referred to as a carbonate sensor. Carbonate gas is a byproduct of battery cells being abused, such as thermal abuse or electrical abuse. An electrolyte in a battery cell may generate carbonate gas in response to the abuse. The gas sensor may be placed close by the battery cell to detect carbonate gas. The gas sensor will thus generate a signal responsive to the change in impedance of the polymer.

In various embodiments, the gas sensor may be configured to provide an output signal that is a binary output signal indicative of when a target gas is above or below a threshold. A device may utilize the gas sensor a switch, which may be used to generate and/or trigger an alarm, alert, or other signal transmission associated with the target gas in the vicinity of the sensor crossing a conductance threshold.

In various embodiments, the gas sensor may be configured to provide a digital output signal and/or an analog output signal associated with the concentration of the target gas. The gas sensor may be able to sense the concentration of the target gas an environment and be calibrated to generate such output signal(s). In various embodiments, such output signal(s) may be a summation of or associated with the summation of one or more electrode output signals.

### Exemplary Systems, Apparatuses, and Methods

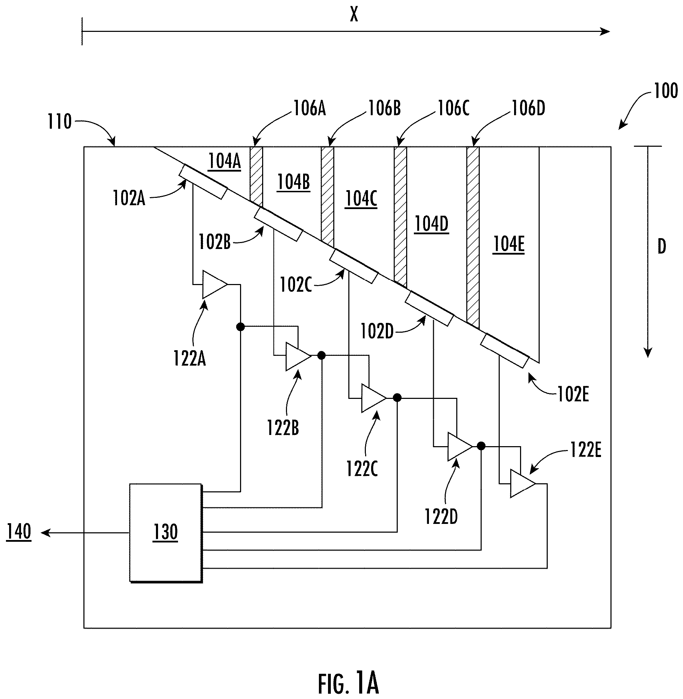

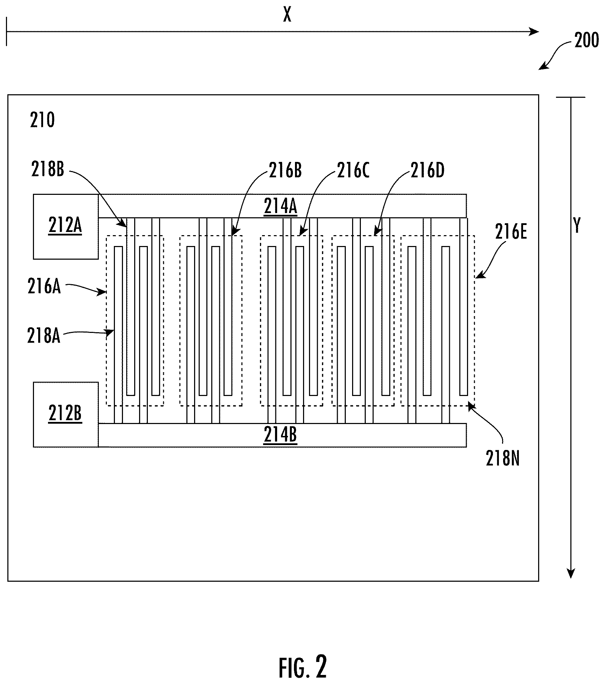

FIG. 1A illustrates a cross-section view of an exemplary block diagram of a gas sensor in accordance with one or more embodiments of the present disclosure. The cross-section view of a first dimension X and a depth D. Not illustrated is a third dimension Y, which will be appreciated is into or out of the illustration. This third dimension Y is illustrated in FIG. 2. Thus the gas sensor 100 illustrated is a 3D gas sensor and, as described herein, is a 3D MEMS gas sensor.

The gas sensor 100 includes a plurality of electrodes 102 (e.g., 102A, 102B, 102C, 102D, 102E) that are located below a respective one of a plurality of polymer coatings 104 (e.g., 104A, 104B, 104C, 104D, 104E). Electrode 102A is below polymer coating 104A, electrode 102B is below polymer coating 102B, and so on. While only five electrode 102 and polymer coating 104 pairs are illustrated, it is appreciated that there may be more, or less, than five of such pairs. An electrode 102 may be made of, for example, gold.

In various embodiments, each of the polymer coatings 104 may be separated by a sidewall 106. For example, a first polymer coating 104A and a second polymer coating 104B may be separated by a first sidewall 106A. As illustrated, each of sidewalls 106A, 106B, 106C, and 106D separate two adjacent polymer coatings 104. Each sidewall 106 may be made of a nonreactive dielectric isolator material to insulate each polymer coating 104 from the adjacent polymer coating(s) 104. In various embodiments, the sidewall 106 may be made of silicon dioxide.

Each electrode 102 (e.g., 102A) is located at a different depth (D) and, thus, the associated polymer coating 104 (e.g., 104A) has a different depth from other polymer coatings 104 (e.g., 104B). During manufacturing the gas sensor 100, the polymer coating 104 is deposited onto the electrode(s) 102 with the deposition(s) being of different depths D.

In various embodiments, the electrodes 102 each at different depths D will have each electrode 102 receive an electrical signal conducted through the polymer coating when the concentration of a target gas at the sensing surface 110 is sufficient to cause an associated polymer coating 104 begin lowering its impedance. In various embodiments, depending on the concentration of the gas present, the electrical signal conducted will change. A low concentration gas will have minimal change(s) in higher depth of polymer. There may be a measurable change in the lower depth of polymer. In various embodiments, the lower depths of polymer may get saturated with the available concentration and the electrode(s) 102 at the next depth will generate an electrical signal that may provide an electrical signal that is collected for measuring the concentration. In various embodiments, the electrical signals output by electrodes 102 of consecutive multiple depths may be used to calculate an interim concentration(s).

For example, at a first concentration of gas that is low, only the polymer coating 104A would have its impedance change from high to low to allow for electrode 102A to receive a signal through the polymer coating 104A. As the concentration in the target gas increases, each of the other polymer coatings 104B to 104E will begin having their respective impedances lowered at different concentrations of the target gas. In various embodiments, controlling for the deposition depth of the polymer coating 104 allows a gas sensor 100 to be configured and/or calibrated to generate electrode output signals associated with one or more concentrations of a target gas.

In various embodiments, the calibration may provide for output signal(s) associated with gas concentration. In various embodiments, a first electrode (e.g., 102A) may be configured to have a step response to 100ppm concentration of a gas and a second electrode (e.g., 102B) may be configured for a step response at 200ppm concentration of the gas. During operation in an exemplary concentration of gas of 150ppm, which is between 100ppm and 200 ppm, the first electrode (e.g., 102A) has a step response (e.g., 102A) to the concentration and the second electrode (e.g., 102B) has a linear response for the 150 ppm concentration. The amplified signal of the first electrode (e.g., 102A) is associated with a concentration of 100 ppm. The amplified signal of the second electrode (e.g., 102B) is generated based on a linearity curve and indicative of a value between 100ppm and 200ppm, which is calibrated for 150ppm. The gas sensor 100 may be calibrated for not just step points associated with saturation of a coating 104 but also for points between steps, which may be referred to as interim points. In various embodiments, the calibration may be resolved through one or more data processing operations that identifies the interim points and generates an output signal associated with the concentrations.

In various embodiments, a polymer coating 104 may be referred to as a polymer segment. While FIG. 1A illustrates that each of the polymer coatings 104A-104E are deposited along a downward ramping slope to illustrate the different depths, it will be appreciated that a polymer coating 104 may be deposited with a flat bottom or another shape. Depending on the amount of concentrations of a polymer coating 104 over an electrode 102 in a particular depth be associated with the concentration or amount of a target gas that is required to lower the impedance of the polymer. It will also be appreciated that the depths of the different polymer coatings 104 do not need to linearly decrease and may distributed throughout (e.g., along the X axis) in a varying order of depth (D).

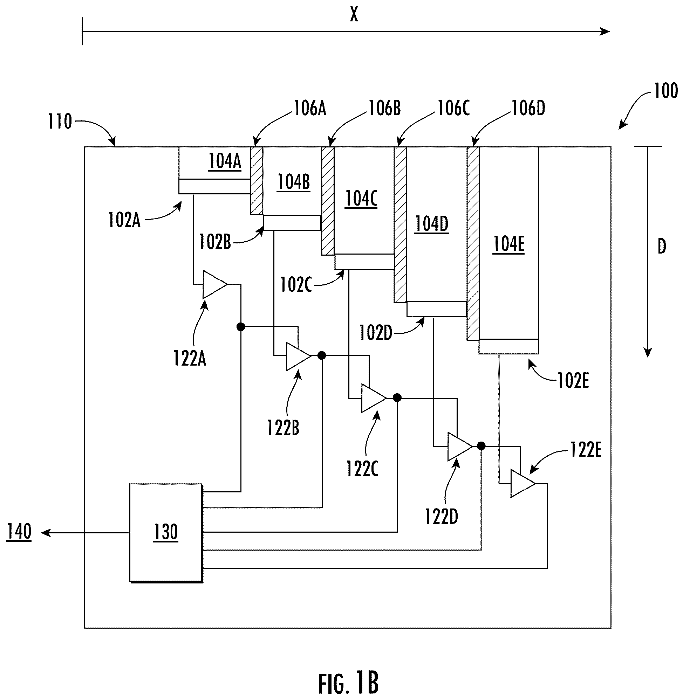

FIG. 1B illustrates a cross-section view of a second exemplary block diagram of a gas sensor in accordance with one or more embodiments of the present disclosure. While FIG. 1A illustrated a ramp arrangement for the electrodes 102, each electrode 102 may be arranged in a step configuration as illustrated in FIG. 1B.

In various embodiments, a power supply will be present that provides a signal to one or more electrodes along the sensing surface 110. The one or more electrodes may be used to provide a polymer coating input signal that will be transmitted through the polymer coating 104 when the impedance has fallen sufficient to conduct the polymer coating input signal. Thus an electrical signal may be transmitted through the polymer coating 104 and received by the electrode 104. The electrode 104 will then generate an electrode output signal that is transmitted out the electrode 104.

For example, when the polymer coating input signal is a voltage signal is a voltage signal then when there is a high impedance of the polymer then the polymer coating 104 may act as an open circuit. When the polymer coating 104 has its impedance change to be lower, the polymer coating 104 may conduct the polymer coating input signal of the voltage signal. Thus the voltage at the electrode will increase as the concentration or exposure increases.

Various embodiments of the polymer coating may also change impedance based not only on the instant concentration of a target gas but the concentration of a target gas over time. For example, various embodiments exposed to a constant concentration of a target gas over time may have a polymer coating 104 lower impedance as the polymer coating 104 may be saturated with the target gas over time.

In various embodiments, as a concentration of a target gas causes the polymer of the polymer coating 104 to become conductive via the change in impedance, thus allowing a current to flow through the polymer coating 104 and to an electrode 102, the electrode output signal may be provided to an amplifier 122 (e.g., 122A, 122B, 122C, 122D, 122E). The amplifier may be, for example, an op amp that amplifier an amplifier input signal to generate an amplifier output signal.

In various embodiments, a power supply may provide a voltage supply to one or more amplifiers 122. Alternatively or additionally, the amplifier output signal may of a first amplifier may provide a voltage supply to a second amplifier. As illustrated in FIG. 1A, this may be repeated with a first amplifier 122A providing a supply to amplifier 122B, which provides a supply to amplifier 122C, which provides a supply to amplifier 122D, which provides a supply to amplifier 122E.

Each amplifier output signal may be provided to a summation circuit 130. In various embodiments, the summation circuit 130 may be summer that adds together the different amplifier output signals. In various embodiments, the summation circuit 130 may include a processor and/or electrical circuitry, such as a multiplexer. The summation circuit 130 may generate a sensor output signal 140 representative of the gas concentration of the target gas based on the multiple inputs of the amplifier output signals from the amplifiers 122. In various embodiments, the summation circuit 130 may generate an alarm, alert, or transmission associated with one or more concentrations. The sensor output signal 140 may be a digital signal or an analog signal. This may include calibrating the summation circuit 130 for the sensor output signal 140 to be associated with different concentrations (e.g., a 0-5V signal with variations associated with different concentrations, a 4-20 milliamp signal with different currents associated with different concentrations, a data packet containing a digital value of the concentration, or the like).

FIG. 2 illustrates a top view of an exemplary block diagram of a gas sensor in accordance with one or more embodiments of the present disclosure. In various embodiments, a gas sensor 200 may include a sensing surface 210 that includes a first terminal 212A and a second terminal 212B. The first terminal 212A may be electrically connected to a first bus 214A. The second terminal 212B may be electrically connected to a second bus 214B. Each of the first bus 214A and the second bus 214B may be electrically collected to a plurality of interdigital electrodes 218 (e.g., 218A-218N) that run parallel or close to each other without touching. One or more portions of the sensing surface 210 may be coated with the polymer coating to separate and, thus, cover the space between the interdigital electrodes 218. The interdigital electrodes may be grouped into pairs, such as a first electrode 218A and second electrode 218B. Alternatively or additionally, the interdigital electrode pairs may be grouped into groups, such as a first electrode group 216A, a second electrode group 216B, a third electrode group 216C, a fourth electrode group 216D, and a fifth electrode group 216E. It will be appreciated more than five groups may be utilized.

In various embodiments, the amount of polymer coating between an electrode pair (e.g., 218A and 218B) may be of a first width (X). In various embodiments, the amount of polymer coating between each of the interdigital electrodes in an electrode grouping 216 (e.g., 216) may be of the same width in the X direction while different from the width in a different group 216 (e.g., 216B). As illustrated, the width between interdigital electrodes of groups 216 increases from narrowest in electrode group 216A to widest in group 216E.

In various embodiments, the first terminal 212A may receive an input signal. When a target gas is present the polymer coating between the interdigital electrodes 218 may become conductive as the concentration of the gas increases. At a first concentration only the polymer coating associated with the first electrode group 216A may be conductive and the input signal may be conducted from the first terminal 212A, through the bust 214A, through the interdigital electrodes 218 of the first electrode group 216A, through the second bus 214B, and to the second terminal 212B. As the gas concentration increases or the time with a concentration increases, the other electrode groupings 216B, 216C, 216D, and 216E may respectively begin conducting in order as their associated polymer coatings respectively change to lower impedances.

While there are illustrated with 5 electrode groupings 216 with four interdigital electrodes each (i.e., two electrodes connected to 214A and two electrodes connected to 214B), it will be appreciated there may be many more electrodes. For example, in various embodiments there may be 92 pairs of interdigital electrode pairs, which has 184 individual electrodes. In various embodiments, there may be 100 interdigital electrode pairs for each of the electrode groupings 216.

While FIG. 2 illustrates embodiments of the present disclosure utilizing a 2 dimensional sensing surface 210, it will be appreciated that by utilizing a single of terminal 212, a bus 214, and/or one or more electrodes 218, a signal may be supplied along a sensing surface 110 of FIG. 1A.

It will also be appreciated that while the interdigital electrode pairs 218 are illustrated in straight lines parallel in the Y direction, various embodiments of the present disclosure may include electrode pairs separated by polymer coating(s) that are in other varied patterns without departing from the present disclosure.

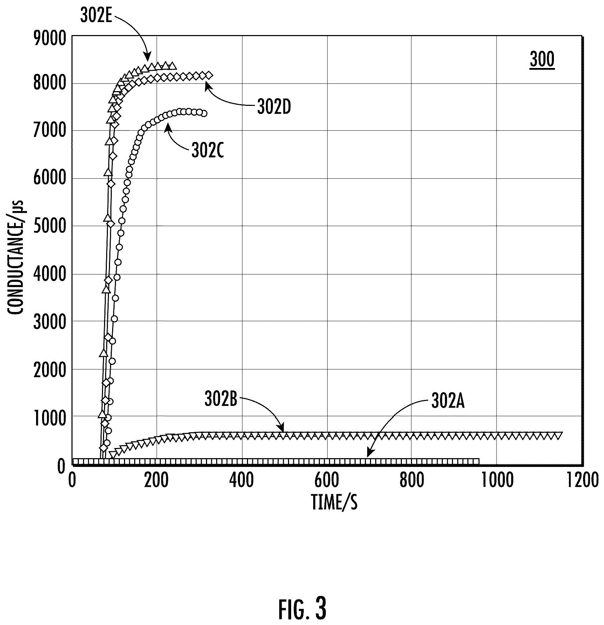

FIG. 3 illustrates a flowchart of example operations for sensing a gas in accordance with one or more embodiments of the present disclosure.

At operation 302, providing a gas sensor with a plurality of electrodes, wherein each electrode is covered with a polymer coating. For example, a gas sensor (e.g., 100, 200) in accordance with one or more embodiments described herein may be provided.

At operation 304, changing, when exposed to a concentration of a target gas, the impedance of one or more of the polymer coatings. When the gas sensor is exposed the target gas in a sufficient concentration then one or more of the polymer coatings will change impedance to a lower impedance.

At operation 306, conducting one or more signals through the polymer coating(s) to the electrodes. When the impedance is lowered, one or more signals are conducted through the one or more polymer coatings with lowered impedance.

At operation 308, amplifying the one or more signals conducted through the electrodes to generate one or more amplified signals. In various embodiments, the one or more polymer coatings may each be associated with an amplifier electrically connected to an electrode that receives the signal(s) conducted through the conducting polymer coating. In various embodiments, each electrode may havea different output response for a different concentration of gas, which may be calibrated to provide concentration data. The signals for reach respective polymer coating may be separately amplified. The amplification may be configured to have an amplified output be configured or calibrated to be at a distinct current and/or voltage associated with the respective polymer coating and, thus, concentration of gas.

At operation 310, generating an output signal based on the one or more amplified signals. The gas sensor may generate an output signal based on the one or more amplified signals. In various embodiments, the one or more amplified signals may be provided to a summation circuit 130 that may sum the amplified signals. In various embodiments, the output signal may be an analog and/or digital signal.

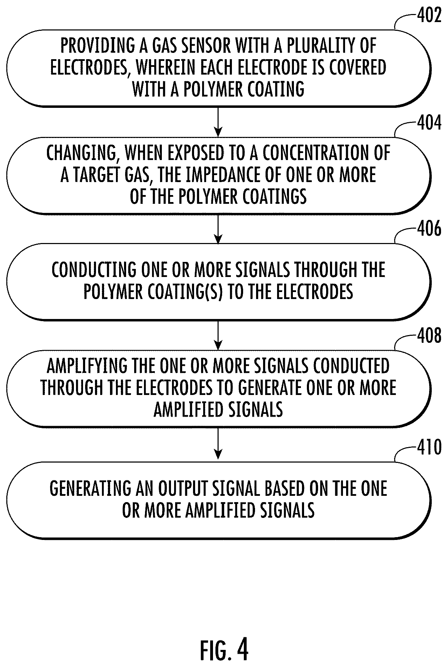

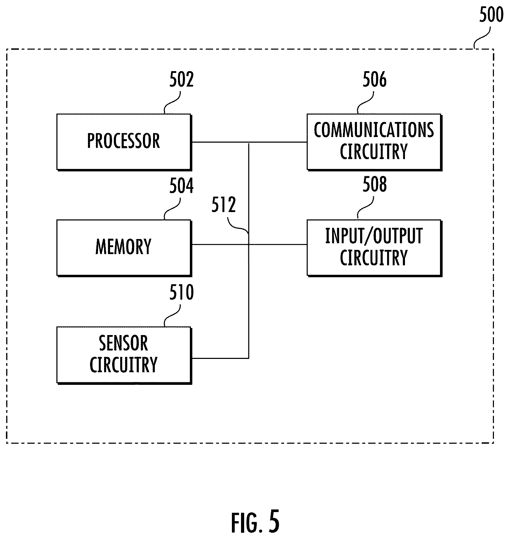

FIG. 4 illustrates an exemplary block diagram of a device in accordance with one or more embodiments of the present disclosure. Exemplary embodiments of the device 400 may include, but are not limited to, switches, gas detectors, alarms, energy storage systems, battery systems, battery protection systems, battery monitors, electric vehicles. The device 400 illustrated includes a processor 402, memory 404, communications circuitry 406, input/output circuitry 408, and sensor circuitry 410. A bus 412 may connect the processor 402, the memory 404, the communications circuitry 406, the input/output circuitry 408, and the sensor circuitry 410.

The processor 402, although illustrated as a single block, may be comprised of a plurality of components and/or processor circuitry. The processor 402 may be implemented as, for example, various components comprising one or a plurality of microprocessors with accompanying digital signal processors; one or a plurality of processors without accompanying digital signal processors; one or a plurality of coprocessors; one or a plurality of multi-core processors; processing circuits; and various other processing elements. The processor may include integrated circuits, such as ASICs, FPGAs, systems-on-a-chip (SoC), or combinations thereof. In various embodiments, the processor 402 may be configured to execute applications, instructions, and/or programs stored in the processor 402, memory 404, or otherwise accessible to the processor 402. When executed by the processor 402, these applications, instructions, and/or programs may enable the execution of one or a plurality of the operations and/or functions described herein. Regardless of whether it is configured by hardware, firmware/software methods, or a combination thereof, the processor 402 may comprise entities capable of executing operations and/or functions according to the embodiments of the present disclosure when correspondingly configured.

The memory 404 may comprise, for example, a volatile memory, a non-volatile memory, or a certain combination thereof. Although illustrated as a single block, the memory 404 may comprise a plurality of memory components. In various embodiments, the memory 404 may comprise, for example, a random access memory, a cache memory, a flash memory, a hard disk, a circuit configured to store information, or a combination thereof. The memory 404 may be configured to write or store data, information, application programs, instructions, etc. so that the processor 402 may execute various operations and/or functions according to the embodiments of the present disclosure. For example, in at least some embodiments, a memory 404 may be configured to buffer or cache data for processing by the processor 402. Additionally or alternatively, in at least some embodiments, the memory 404 may be configured to store program instructions for execution by the processor 402. The memory 404 may store information in the form of static and/or dynamic information. When the operations and/or functions are executed, the stored information may be stored and/or used by the processor 402.

The communication circuitry 406 may be implemented as a circuit, hardware, computer program product, or a combination thereof, which is configured to receive and/or transmit data from/to another component or apparatus. The computer program product may comprise computer-readable program instructions stored on a computer-readable medium (e.g., memory 404) and executed by a processor 402. In various embodiments, the communication circuitry 406 (as with other components discussed herein) may be at least partially implemented as part of the processor 402 or otherwise controlled by the processor 402. The communication circuitry 406 may communicate with the processor 402, for example, through a bus 412. Such a bus 412 may connect to the processor 402, and it may also connect to one or more other components of the processor 402. The communication circuitry 406 may be comprised of, for example, transmitters, receivers, transceivers, network interface cards and/or supporting hardware and/or firmware/software, and may be used for establishing communication with another component(s), apparatus(es), and/or system(s). The communication circuitry 406 may be configured to receive and/or transmit data that may be stored by, for example, the memory 404 by using one or more protocols that can be used for communication between components, apparatuses, and/or systems.

The input/output circuitry 408 may communicate with the processor 402 to receive instructions input by an operator and/or to provide audible, visual, mechanical, or other outputs to an operator. The input/output circuitry 408 may comprise supporting devices, such as a keyboard, a mouse, a user interface, a display, a touch screen display, lights (e.g., warning lights), indicators, speakers, and/or other input/output mechanisms. The input/output circuity 408 may comprise one or more interfaces to which supporting devices may be connected.

The sensor circuitry 410 may include a gas sensor (e.g., 100, 200). In various embodiments, the sensor circuitry 410 may include one or more gas sensors. In various embodiments, the sensor circuitry 410 may also include a power supply and/or signal generator. In various embodiments, the sensor circuitry 410 may be configured to receive an input signal to provide to one or more electrodes that will be conducted through a polymer coating (e.g., 104) when a gas concentration is above a first threshold. The sensor circuitry 410 may be configured to provide an output signal (e.g., 140) based on concentration of a target gas.

It should be readily appreciated that the embodiments of the systems, apparatuses, and methods described herein may be configured in various additional and alternative manners in addition to those expressly described herein.

### Conclusion

Operations and/or functions of the present disclosure have been described herein, such as in flowcharts. As will be appreciated, computer program instructions may be loaded onto a computer or other programmable apparatus (e.g., hardware) to produce a machine, such that the resulting computer or other programmable apparatus implements the operations and/or functions described in the flowchart blocks herein. These computer program instructions may also be stored in a computer-readable memory that may direct a computer, processor, or other programmable apparatus to operate and/or function in a particular manner, such that the instructions stored in the computer-readable memory produce an article of manufacture, the execution of which implements the operations and/or functions described in the flowchart blocks. The computer program instructions may also be loaded onto a computer, processor, or other programmable apparatus to cause a series of operations to be performed on the computer, processor, or other programmable apparatus to produce a computer-implemented process such that the instructions executed on the computer, processor, or other programmable apparatus provide operations for implementing the functions and/or operations specified in the flowchart blocks. The flowchart blocks support combinations of means for performing the specified operations and/or functions and combinations of operations and/or functions for performing the specified operations and/or functions. It will be understood that one or more blocks of the flowcharts, and combinations of blocks in the flowcharts, can be implemented by special purpose hardware-based computer systems which perform the specified operations and/or functions, or combinations of special purpose hardware with computer instructions.

While this specification contains many specific embodiments and implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

While operations and/or functions are illustrated in the drawings in a particular order, this should not be understood as requiring that such operations and/or functions be performed in the particular order shown or in sequential order, or that all illustrated operations be performed, to achieve desirable results. In certain circumstances, operations and/or functions in alternative ordering may be advantageous. In some cases, the actions recited in the claims may be performed in a different order and still achieve desirable results. Thus, while particular embodiments of the subject matter have been described, other embodiments are within the scope of the following claims.

While this detailed description has set forth some embodiments of the present invention, the appended claims cover other embodiments of the present invention which differ from the described embodiments according to various modifications and improvements.

## Claims

1. A 3D MEMS gas sensor comprising:
a plurality of electrodes; and
a plurality of polymer coatings, wherein each of the plurality of polymer coatings is deposited on top of one of one of the plurality of electrodes, and wherein each of the plurality of polymer coatings has a different coating depth.

2. The 3D MEMS gas sensor of claim 1, wherein each polymer coating of the plurality polymer coatings is comprised of a polymer that changes impedance based on a concentration of a first gas.

3. The 3D MEMS gas sensor of claim 2, wherein the 3D MEMS gas sensor is configured to generate an output signal that varies based on the concentration of the first gas.

4. The 3D MEMS gas sensor of claim 2, wherein the first gas is a carbonate gas.

5. The 3D MEMS gas sensor of claim 1, wherein each of the plurality of polymer coatings is separated from one or more adjacent polymer coatings by one or more sidewalls.

6. The 3D MEMS gas sensor of claim 5, wherein each of the one or more sidewalls is comprised of a dielectric material.

7. The 3D MEMS gas sensor of claim 1 further comprising a plurality of amplifiers, and wherein each of the plurality of electrodes is electrically connected to one of the plurality of amplifiers, and wherein each of the plurality of amplifiers is configured to amplify an electrode signal received from the associated electrode.

8. The 3D MEMS gas sensor of claim 7, wherein a first amplifiers of the plurality of amplifiers is electrically connected to a second amplifier of the plurality of amplifiers to provide a supply voltage to the second amplifier.

9. The 3D MEMS gas sensor of claim 7, wherein each of the plurality of amplifiers comprise an amplifier output; and
wherein each of the amplifier outputs is electrically connected to a summation circuit configured to generate a summation circuit output signal based on one or more amplifier output signals received from the plurality of amplifiers.

10. The 3D MEMS gas sensor of claim 9, wherein the summation circuit is configured to generate a summation circuit output signal of a voltage associated with the concentration of the first gas.

11. A method of gas sensing comprising:
providing a 3D MEMS gas sensor comprising:
a plurality of electrodes; and
a plurality of polymer coatings, wherein each of the plurality of polymer coatings is deposited on top of one of one of the plurality of electrodes, and wherein each of the plurality of polymer coatings has a different coating depth;
exposing the 3D MEMS gas sensor to a first gas; and
generating an output signal when a concentration of the first gas is above a first threshold.

12. The method of gas sensing of claim 11, wherein each polymer coating of the plurality polymer coatings is comprised of a polymer that changes impedance based on the concentration of the first gas.

13. The method of gas sensing of claim 12, wherein the output signal changes when the concentration of the first gas is above a second threshold.

14. The method of gas sensing of claim 11, wherein the 3D MEMS gas sensor further comprises:
a plurality of amplifiers;
wherein each of the plurality of electrodes is electrically connected to one of the plurality of amplifiers; and
wherein each of the plurality of amplifiers is configured to amplify an electrode signal received from the associated electrode.

15. The method of gas sensing of claim 14, wherein a first amplifiers of the plurality of amplifiers is electrically connected to a second amplifier of the plurality of amplifiers to provide a supply voltage to the second amplifier.
